# EUROPEAN PATENT SPECIFICATION

(11) **EP 2 968 114 B1**
(45) Date of publication and mention of the grant of the patent: **12.06.2019**
(21) Application number: 14716676.3
(22) Date of filing: 10.03.2014
(51) Int. Cl.: A61K 9/00, A24B 13/00, B65B 1/04

(54) **METHOD AND APPARATUS FOR DIFFERENTIATING ORAL POUCH PRODUCTS**
VERFAHREN UND VORRICHTUNG ZUR UNTERSCHEIDUNG ORALER BEUTELPRODUKTE
PROCÉDÉ ET APPAREIL POUR DIFFÉRENTIER DES PRODUITS ORAUX EN SACHET

(30) Priority: 11.03.2013 US 201313792926
(43) Date of publication of application: 20.01.2016
(73) Proprietor: Niconovum USA, Inc., Winston-Salem, North Carolina 27101 (US)
(72) Inventor: REDDICK, Edwin Matthew, Clemmons, North Carolina 27012 (US); ARMSTRONG, Stephen Taylor, Kernersville, North Carolina 27284 (US)
(74) Representative: Hoeger, Stellrecht & Partner Patentanwälte mbB
(86) International application number: PCT/US2014/022646
(87) International publication number: WO 2014/164509

(56) References cited:
- EP-A1- 2 428 126
- EP-A1- 2 454 954
- US-A1- 2010 300 465

## Description

### FIELD OF INVENTION

The present disclosure relates to an oral nicotine-containing pharmaceutical pouch product wherein identifying information related to the product is provided on the pouch, and methods of forming such products.

### BACKGROUND

Central nervous system (CNS) conditions, diseases, or disorders can be drug induced; can be attributed to genetic predisposition, infection or trauma; or can be of unknown etiology. They comprise neuropsychiatric disorders, neurological diseases and mental illnesses; and include neurodegenerative diseases, behavioral disorders, cognitive disorders and cognitive affective disorders. The clinical manifestations of several CNS conditions, diseases or disorders have been attributed to CNS dysfunction (i.e., disorders resulting from inappropriate levels of neurotransmitter release, inappropriate properties of neurotransmitter receptors, and/or inappropriate interaction between neurotransmitters and neurotransmitter receptors).

Nicotinic compounds, such as nicotine, are capable of affecting nicotinic acetylcholinergic receptors (nAChRs). Subtypes of nAChRs exist in both the CNS and the peripheral nervous system (PNS), but the distribution of subtypes is heterogeneous. For instance, certain subtypes which are predominant in vertebrate brain, others predominate at the autonomic ganglia, and others predominate at neuromuscular junction. Activation of nAChRs by nicotinic compounds results in neurotransmitter release. See, for example, Dwoskin et al., Exp. Opin. Ther. Patents, 10: 1561-1581 (2000); Schmitt et al., Annual Reports in Med. Chem. 35: 41-51 (2000); Huang et al., J. Am. Chem. Soc., 127: 14401-14414 (2006); Arneric et al., Biochem. Pharmacol., 74: 1092-1101 (2007) and Millar, Biochem. Pharmacol., 78: 766-776 (2009).

It has been suggested that administration of nicotine, and other nicotinic compounds, can result in various pharmacological effects. See, for example, US Pat. Nos. 5,583,140 to Bencherif et al.; 5,723,477 to McDonald et al.; 7,001,900 to Jacobsen et al.; 7,135,484 to Dart et al. and 7,214,686 to Bencherif et al.; and US Pat. Pub. No. 2010/0004451 to Ahmad et al. As a result, it has been suggested that nicotine, and other nicotinic compounds, can exhibit utility in the treatment of a wide variety of conditions, diseases, and disorders, including those that affect the CNS. Additionally, administration of nicotine and nicotinic compounds has been proposed for treatment of certain other conditions, diseases, and disorders. See, for example, US Pat. Nos. 5,604,231 to Smith et al.; 5,811,442 to Bencherif et al.; 6,238,689 to Rhodes et al. Furthermore, administration of nicotine has been employed in an effort to help cigarette smokers quit smoking (i.e., as a smoking cessation aid). For example, nicotine has been an active ingredient of various types of so-called "nicotine replacement therapy" or "NRT" products. See, for example, background art discussed in US Pat. App. Ser. No. 13/278,877 to Borschke et al, filed October 21, 2011.

One particular method that has been employed to provide oral administration of nicotine is through the use of nicotine-containing snuff-type forms in pouches or sachets (e.g., US Pat. No. 4,907,605 to Ray et al. and US Pat. Pub. No. 2009/0293895 to Axelsson et al.). Various products intended for oral use employ moisture permeable pouches or sachets. During use, those pouches or sachets are inserted into the mouth of the user, and water soluble components contained within those pouches or sachets are released as a result of interaction with saliva.

Certain commercially available smokeless tobacco products, such as products commonly referred to as "snus," comprise ground tobacco materials incorporated within sealed pouches. Representative types of snus products have been manufactured in Europe, particularly in Sweden, by or through companies such as Swedish Match AB (e.g., for brands such as General, Ettan, Goteborgs Rape and Grovsnus); Fiedler & Lundgren AB (e.g., for brands such as Lucky Strike, Granit, Krekt and Mocca); JTI Sweden AB (e.g., for brands such as Gustavus) and Rocker Production AB (e.g., for brands such as Rocker). Other types of snus products have been commercially available in the U.S.A. through companies such as Philip Morris USA, Inc. (e.g., for brands such as Marlboro Snus); U.S. Smokeless Tobacco Company (e.g., for brands such as SKOAL Snus) and R. J. Reynolds Tobacco Company (e.g., for brands such as CAMEL Snus). See also, for example, Bryzgalov et al., 1N1800 Life Cycle Assessment, Comparative Life Cycle Assessment of General Loose and Portion Snus (2005).

Various types of snus products, as well as components for those products and methods for processing components associated with those products, have been proposed. See, for example, US Pat. Nos. 8,067,046 to Schleef et al. and 7,861,728 to Holton, Jr. et al.; US Pat. Pub. Nos. 2004/0118422 to Lundin et al.; 2008/0202536 to Torrence et al.; 2009/0025738 to Mua et al.; 2011/0180087 to Gee et al.; 2010/0218779 to Zhuang et al.; 2010/0294291 to Robinson et al.; 2010/0300465 to Zimmermann; 2011/0061666 to Dube et al.; 2011/0303232 to Williams et al.; 2012/0067362 to Mola et al.; 2012/0085360 to Kawata et al.; 2012/0103353 to Sebastian et al. and 2012/0247492 to Kobal et al.; and PCT Pub. Nos. WO 05/063060 to Atchley et al. and WO 08/56135 to Onno. In addition, certain quality standards associated with snus manufacture have been assembled as a so-called GothiaTek standard. Furthermore, various manners and methods useful for the production of snus types of products have been proposed. See, for example, US Patent Nos. 4,607,479 to Linden and 4,631,899 to Nielsen; and US Pat. Pub. Nos. 2008/0156338 to Winterson et al.; 2010/0018539 to Brinkley et al.; 2010/0059069 to Boldrini; 2010/0071711 to Boldrini; 2010/0101189 to Boldrini; 2010/0101588 to Boldrini; 2010/0199601 to Boldrini; 2010/0200005 to Fallon; 2010/0252056 to Gruss et al.; 2011/0284016 to Gunter et al.; 2011/0239591 to Gruss et al.; 2011/0303511 to Brinkley et al.; 2012/0055493 to Novak III et al. and 2012/0103349 to Hansson et al.; and PCT Pub. Nos. WO 2008/081341 to Winterson et al. and WO 2008/146160 to Cecil et al. Additionally, snus products can be manufactured using equipment such as that available as SB 51-1/T, SBL 50 and SB 53-2/T from Merz Verpackungmaschinen GmBH.

Certain types of products employing pouches or sachets that contain tobacco substitutes (or combinations of tobacco and tobacco substitutes) also have been proposed. See, for example, US Pat. Nos. 5,167,244 to Kjerstad and 7,950,399 to Winterson et al.; and US Pat. Pub. Nos. 2005/0061339 to Hansson et al.; 2011/0041860 to Essen et al. and 2011/0247640 to Beeson et al.

Certain types of product employing pouches or sachets have been employed to contain nicotine, such as those used for nicotine replacement therapy (NRT) types of products (e.g., a pharmaceutical product distributed under the tradename ZONNIC® by Niconovum AB). See also, for example, the types of pouch materials and nicotine-containing formulations set forth in US Pat. No. 4,907,605 to Ray et al.; US Pat. Pub. Nos. 2009/0293895 to Axelsson et al. and 2011/0268809 to Brinkley et al.; and PCT Pub. Nos. WO 2010/031552 to Axelsson et al. and WO 2012/134380 to Nilsson.

EP 2 454 954 A1 discloses an oral tobacco product with a mixture containing, as its main constituent, tobacco particles made from a tobacco material, a pouch filled with the mixture, and product information relating to the mixture and provided on an outer surface of the pouch.

EP 2 428 126 A1 discloses a smokeless oral tobacco product comprising a permeable pouch and smokeless tobacco. The permeable pouch comprises a woven polylactide material.

US 2010/300465 A1 discloses a pouched, oral tobacco product including a porous membrane having a liner and an inner filling material. The inner filling material includes loose, fibrous tobacco material and tobacco beads formed from tobacco fines and dust which are too small to be included in traditional pouched tobacco products.

It would be desirable to provide an oral nicotine-containing pharmaceutical product wherein identifying information related to the product is provided to a user of the product.

### SUMMARY OF THE INVENTION

The present invention relates to an oral pouch enclosing a nicotine-containing pharmaceutical composition in accordance with claim 1, wherein product information related to the materials enclosed in the pouch is provided on the pouch or within the pouch. Accordingly, the oral pouch can be visually distinguished from other similar pouch-type oral pouch products and can also stimulate a user's purchasing interest.

Various embodiments of a nicotine-containing pharmaceutical product configured for insertion into the mouth of a user of that product comprise an outer water-permeable pouch containing a nicotine-containing pharmaceutical composition and product identifying information relating to the nicotine-containing pharmaceutical composition presented such that said product identifying information is discernible to a user of the product upon visual inspection of the product and which enables the user to differentiate multiple nicotine-containing pharmaceutical products. In various embodiments, the product identifying information can comprise one or more alphanumeric characters, designs, and colors associated with the nicotine-containing pharmaceutical product. The product identifying information is imprinted on the outer water-permeable pouch. In some embodiments, product identifying information can be provided by a selection of material (e.g., a material that is different in composition, different in color, different in texture, different in thickness, or different in some other defined property). In some embodiments, the product identifying information is selected from the group consisting of product brand, a company name, a corporate logo, a corporate brand, a marketing message, product strength, active ingredient, product manufacture date, product expiration date, product flavor, product pharmaceutical release profile, weight, product code (e.g., batch code), other product differentiating markings, and combinations thereof.

The product identifying information is imprinted on a seam of the outer water-permeable pouch. In an embodiment, the imprinted product identifying information can identify the strength of the nicotine-containing pharmaceutical composition. The imprinted product identifying information seals the seam of the outer water-permeable pouch. Furthermore, the outer water-permeable pouch can be transparent or translucent such that the colored component of the nicotine-containing pharmaceutical composition can be discernible to a user of the product upon visual inspection of the product.

The present disclosure also relates to a method for manufacturing a nicotine-containing pharmaceutical product in accordance with claim 4. In various embodiments, the method comprises providing a continuous supply of a pouch material, engaging lateral edges of the pouch material such that a longitudinally-extending seam is formed, sealing the longitudinally-extending seam such that a continuous tubular member is formed from the continuous supply of pouch material, inserting a nicotine-containing pharmaceutical composition into the continuous tubular member, subdividing the continuous tubular member into discrete pouch portions such that each pouch portion includes a nicotine-containing pharmaceutical charge, sealing a leading and an end edge of each discrete pouch portion such that an outer water-permeable pouch is formed that encloses the nicotine-containing pharmaceutical charge, and providing product identifying information relating to the nicotine-containing pharmaceutical charge such that said product identifying information is discernible to a user of the product upon visual inspection of the product and which enables the user to differentiate multiple nicotine-containing pharmaceutical products. As described above, the product identifying information can comprise one or more of alphanumeric characters, designs, and colors associated with the nicotine-containing pharmaceutical product.

The product identifying information is imprinted on the outer water-permeable pouch. The various ways of providing product identifying information disclosed above can also be employed in relation to the pouch formation method described herein.

This disclosure is intended to be read holistically such that any separable features or elements of the disclosed invention, in any of its various aspects and embodiments, should be viewed as intended to be combinable unless the context clearly dictates otherwise

### BRIEF DESCRIPTION OF THE DRAWINGS

Having thus described the invention in the foregoing general terms, reference will not be made to the accompanying drawings, which are not necessarily drawn to scale, and wherein:
FIGs. 1A and 1B are front perspective views each illustrating a nicotine-containing pharmaceutical product wherein product identifying information has been printed on an exposed surface of an outer water-permeable pouch;
FIGs. 2A and 2B are front perspective views each illustrating a nicotine-containing pharmaceutical product according to the present invention wherein product identifying information has been imprinted on longitudinally-extending seam of an outer water-permeable pouch;
FIG. 3A is a front perspective view illustrating a nicotine-containing pharmaceutical product wherein product identifying information has been provided by dyeing at least a portion of an outer water-permeable pouch;
FIG. 3B is a front perspective view illustrating a nicotine-containing pharmaceutical product wherein product identifying information has been provided by dyeing at least a portion of a nicotine-containing pharmaceutical composition situated within an outer water-permeable pouch;
FIG. 4A is a front perspective view illustrating a nicotine-containing pharmaceutical product wherein colored granules are blended with a nicotine-containing pharmaceutical composition within an outer water-permeable pouch such that product identifying information is provided;
FIG. 4B is a front perspective view illustrating a nicotine-containing pharmaceutical product wherein a colored strip element is included within an outer water-permeable pouch such that product identifying information is provided;
FIG. 5A is a front perspective view illustrating a nicotine-containing pharmaceutical product wherein a product identifying strip element is woven through an exposed surface of an outer water-permeable pouch such that product identifying information is provided;
FIG. 5B is a front perspective view illustrating a nicotine-containing pharmaceutical product wherein a product identifying strip element is woven through at least one laterally-extending seam of an outer water-permeable pouch such that product identifying information is provided; and
FIG. 6 is a flow chart illustrating the general steps for manufacturing a nicotine-containing pharmaceutical product according to an embodiment of the present invention.

### DETAILED DESCRIPTION

The present invention now will be described more fully hereinafter. This invention may, however, be embodied in many different forms and should not be construed as limited to the embodiments set forth herein; rather, these embodiments are provided so that this disclosure will be thorough and complete, and will fully convey the scope of the invention to those skilled in the art. As used in this specification and the claims, the singular forms "a," "an," and "the" include plural referents unless the context clearly dictates otherwise.

According to the invention, a nicotine-containing pharmaceutical product configured for insertion into the mouth of a user of that product is provided. The product comprises an outer water-permeable pouch, a nicotine-containing pharmaceutical composition situated within the outer water-permeable pouch, and product identifying information relating to the nicotine-containing pharmaceutical composition. The information is presented in such a way that a user of the product can discern the identifying information by visually inspecting the product and thereby differentiate or identify certain nicotine-containing pharmaceutical products. The product identifying information is imprinted (e.g., embossed, debossed, or otherwise pressed) on the outer water-permeable pouch. The product identifying information can be applied through heat and pressure. Roller bars used to press longitudinal and/or lateral seams in a pouch manufacturing process can be modified such that the roller bars are engraved or otherwise patterned to assist in marking a pouch as a seam is pressed. Accordingly, product identifying information are provided during formation of the pouch material as the fabric is manufactured In some embodiments, the product identifying information can identify a product brand, a company name, a corporate logo, a corporate brand, a marketing message, product strength, active ingredient, product manufacture date, product expiration date, product flavor, product pharmaceutical release profile, weight, product code (e.g., batch code), other product differentiating markings, and combinations thereof. As used herein, "product strength" refers to information that gives the user an indication of the amount of active ingredient within the pouch.

Various types of pouch materials and pouch manufacturing techniques are discussed in more detail below. Generally, the products include a powdered or granular nicotine-containing pharmaceutical composition that is disposed within a moisture-permeable container. That is, the nicotine-containing pharmaceutical composition can be contained within a container, such as a pouch or bag, such as the type commonly used for the manufacture of snus types of products (e.g., a sealed, moisture permeable pouch that is sometimes referred to as a "portion"). A representative moisture permeable pouch can be composed of a "fleece" type of material. The container is intended to be placed in the mouth of the user, such that the nicotine-containing pharmaceutical composition within the container may be enjoyed by the user. After the product user is finished using the nicotine-containing pharmaceutical product, the container may be removed from the user's mouth for disposal. In some instances, the container may be manufactured from a water dissolvable or dispersal material, such that the nicotine-containing pharmaceutical composition and the container can be ingested by the user. For example, a pouch type of product similar in shape and form to various embodiments of a nicotine-containing pharmaceutical pouch described herein is commercially available as ZONNIC (distributed by Niconovum AB), and is produced using generally similar pouch material, excipient ingredients and processing conditions used for the manufacture of a pouch useful for embodiments of the nicotine-containing pharmaceutical product described herein. Additionally, pouch type products generally similar in shape and form to various embodiments of a nicotine-containing pharmaceutical pouch product are set forth as snuff bag compositions E-J in Example 1 of PCT WO 2007/104573 to Axelsson et al., which are produced using excipient ingredients and processing conditions that can be used to manufacture nicotine-containing pharmaceutical products as described herein. Adhesives, coatings, colorants, and other ingredients used in products described herein can be generally recognized as safe, non-toxic, ingestible and otherwise suitable for use as a pharmaceutical product.

In various embodiments of the product, product identifying information can be provided. The product indentifying information can include, for example, one or more alphanumeric characters, designs, and colors associated with the nicotine-containing pharmaceutical product. For example, the product identifying information can include a character string such as "ABC," "123", or "AA1." Any combination of alphanumeric characters and/or words can be used to provide product identifying information. In various embodiments the product identifying information can include a pictorial figure or character design. In some embodiments, the product identifying information can include alphanumeric characters in combination with a pictorial figure or character design.

In various embodiments, the product identifying information provided can represent or explicitly state any information regarding the product that a user might find relevant or helpful. For example, without limitation, the product identifying information can indicate the contents of the nicotine-containing pharmaceutical composition, the amount of the nicotine-containing pharmaceutical composition contained within the pouch or the amount of active agent (e.g., nicotine) in the pouch, the name or chemical formula of an additive within the nicotine-containing pharmaceutical composition, a product flavor description, a product brand, a company name, a corporate logo or brand, marketing messages, a dosage or strength of the nicotine-containing pharmaceutical composition, a product manufacture date, a product expiration date, a product pharmaceutical release profile, weight, a product code (e.g., batch code), other product differentiating markings, and a combination of the foregoing. The above-noted types of product identifying information can be presented alphanumerically or through use of images or colors, such as where such images or colors are associated with product identifying information in materials accompanying the product or otherwise available to the product user. For example, various colors can be associated with different product strengths by communicating the association between color and strength in other packaging materials accompanying the product, through a product website, or the like.

Various methods can be employed to provide product identifying information. For example, the product identifying information can be printed on the outer water-permeable pouch. In the present invention, the product identifying information is imprinted on the outer water-permeable pouch. In some embodiments, an additional component (e.g., a colored string, strip, or pellets) is placed inside the outer water-permeable pouch in a way that it is visible to a user of the pouch and thereby enables the user to visually discern the product identifying information. In various embodiments, an additional component (e.g., a colored string or strip) is attached to the outer water-permeable pouch in a way that is visible to a user of the pouch, thereby enabling the user to visually discern the product identifying information. As used herein, attached can refer to product identifying information deposited on the outer water-permeable pouch, adhered to the outer water-permeable pouch, positioned in intimate contact with the outer water-permeable pouch, woven or sewn onto the outer water-permeable pouch, or otherwise attached to the outer water-permeable pouch as is well known in the art. Aspects of the present invention are more fully illustrated by the following examples, which are set forth to illustrate certain aspects of the present invention and are not construed as limiting thereof.

In various embodiments of the nicotine-containing pharmaceutical product, the outer water-permeable pouch is made from a fleece material, as is well known in the art. In some embodiments, product identifying information can be printed onto the fleece material. As used herein, "printing" is a process for transferring text and/or images to a substrate and includes methods such as offset printing, rotogravure, flexography, platen printing, screen printing, inkjet printing, laser printing, and the like. In various embodiments, online printing of the fleece during pouch manufacture can be employed such that the printing step is part of the nicotine-containing pharmaceutical product manufacturing process. Product identifying information can be printed onto the fleece material prior to pouching (i.e., forming the sealed nicotine-containing pharmaceutical product with the nicotine-containing pharmaceutical composition situated inside the outer water-permeable pouch, wherein the outer water-permeable pouch is formed from the fleece material). Offline printing of the fleece during fleece production can be used to print identifying product information onto the fleece material. For example, a secondary offline printing process can be included during the splitting and/or rewind steps of the pouch product manufacturing process. For both online and offline printing, the printing apparatus can be situated and/or calibrated such that the product identifying information can be printed at any location on the nicotine-containing pharmaceutical product.

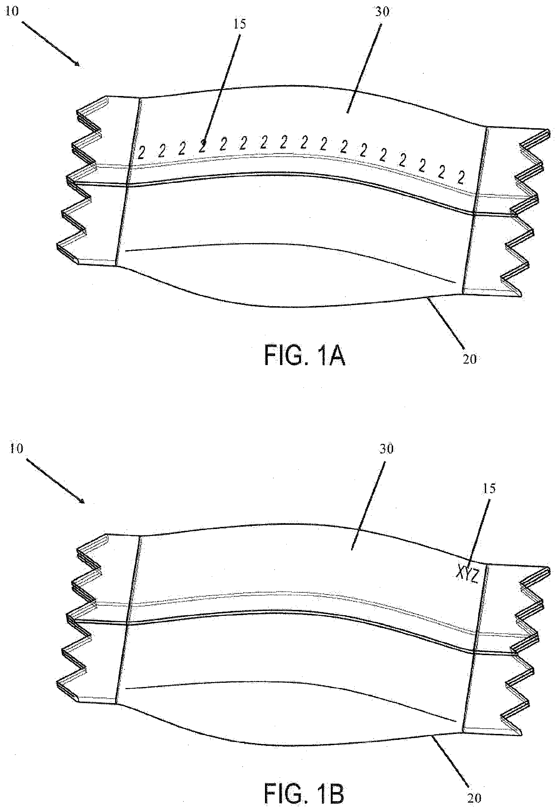

For example, as illustrated in FIG. 1A, product identifying information **15** can be printed on an exposed surface **30** of the nicotine-containing pharmaceutical product **10**. FIG. 1A illustrates product identifying information indicating dosage strength of the nicotine-containing pharmaceutical composition situated within the outer water-permeable pouch **20**, wherein the information is extended along a longitudinal length of the outer water-permeable pouch **20**. As illustrated in FIG. 1B, for example, product identifying information **15** can be printed in any specific location on an exposed surface **30** of the outer water-permeable pouch **20**. FIG. 1B illustrates product identifying information indicating the brand of the nicotine-containing pharmaceutical product **10**, wherein the information is located in the upper right corner of the outer surface of the outer water-permeable pouch **20**. However, the orientation and type of information illustrated herein are not construed as limiting thereof.

In the present invention, the product identifying information is provided by imprinting the outer water-permeable pouch material using a registered imprint device. As used herein, the term "imprinting" refers to either an embossing or a debossing process. Embossing and debossing are processes of pressing paper or other materials into relief using heat and force. Embossing creates a raised impression while debossing creates an indented impression. The finished product will have a three dimensional (i.e., raised or indented) effect. In some embodiments, the fleece material is imprinted during pouch manufacture. For example, the fleece material can be imprinted along a longitudinally-extending seam during the sealing step using a longitudinal heating element. In some embodiments, the fleece material can be imprinted along a laterally-extending seam during the sealing step using imprinting equipment known in the art. In the present invention, roller bars used to press longitudinal and/or lateral seams in a pouch manufacturing process can be modified such that the roller bars can be engraved or otherwise patterned to assist in marking a pouch as a seam is pressed. In this manner, the imprinted product identifying information serves not only a product identification function, but the imprinting also contributes to sealing of the pouch. The fleece material can be imprinted during fleece production in a secondary offline process prior to or after forming the pouch product such that product identifying information is provided at any location and orientation on an exposed surface of the outer water-permeable pouch. According to the invention, the product identifying information is imprinted on the outer water-permeable pouch such that a user can easily view the product identifying information. Any combination of alphanumeric characters, designs, patterns, etc. can be imprinted onto a nicotine-containing pharmaceutical product.

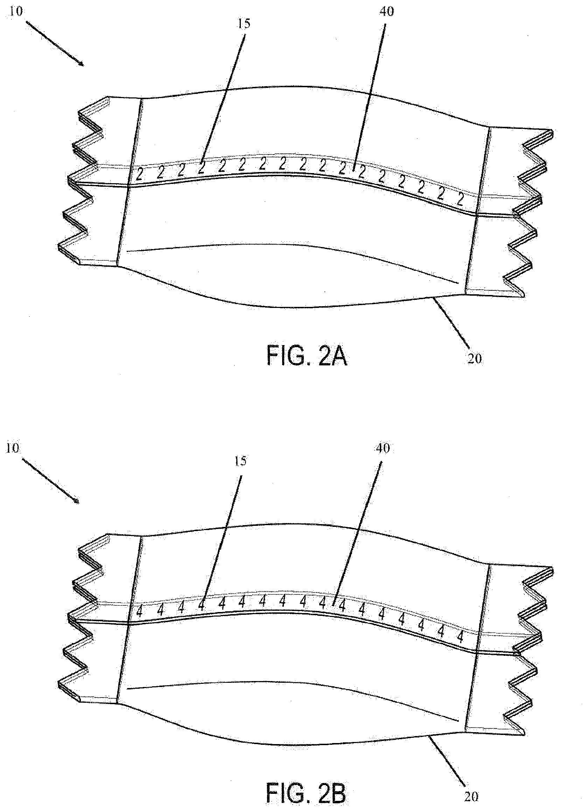

As illustrated in FIGs. 2A and 2B, for example, the product identifying information **15** can be imprinted along a longitudinally-extending seam **40** as the outer water-permeable pouch **20** of the nicotine-containing pharmaceutical product **10** is sealed. Different embodiments of the nicotine-containing pharmaceutical product **10** can be readily identified by specific product identifying information **15.** FIG. 2A, for example, indicates a nicotine-containing pharmaceutical composition comprising 2 mg of nicotine active ingredient. FIG. 2B, for example, indicates a nicotine-containing pharmaceutical composition comprising 4 mg of nicotine active ingredient. In this manner, the product user can immediately visualize the difference in product strength by visual examination of the exterior of the pouch of each product. Any combination of alphanumeric characters, designs, patterns, etc. can be imprinted along the horizontal seam and the orientation and type of information illustrated herein are not construed as limiting thereof.

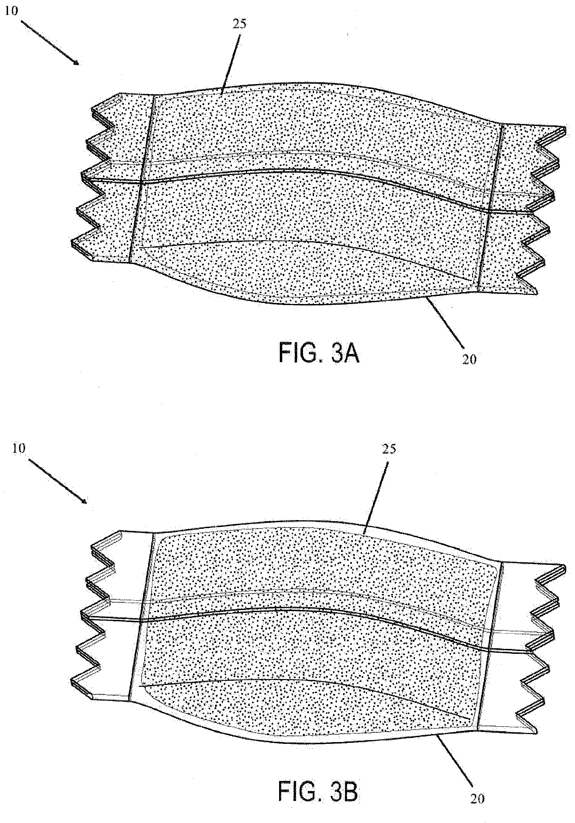

The product identifying information can be provided by using an edible colorant to dye at least a portion of the fleece used to form the outer water-permeable pouch and/or at least a portion of the nicotine-containing pharmaceutical composition. At least a portion of the fleece can by dyed with an edible colorant during fleece production. At least a portion of the fleece can be dyed with an edible colorant during the pouch manufacturing process. For example, as illustrated in FIG. 3A, the outer water-permeable pouch **20** can be formed entirely of fleece that has been dyed with an edible colorant. Various colors can be used to represent information related to the nicotine-containing pharmaceutical composition **25** within the outer water permeable pouch **20**. As illustrated in FIG. 3B, for example, various embodiments can comprise a nicotine-containing pharmaceutical composition **25** that has been dyed with an edible colorant. For example, the colored component of the nicotine-containing pharmaceutical composition can be a colored porous material carrying a nicotinic compound. In various embodiments, the porous carrier can be microcrystalline cellulose ("MCC") carrier wherein the nicotine-containing pharmaceutical composition is at least partly sorbed on MCC and/or is at least partially absorbed into the carrier and/or is at least partially adsorbed onto the carrier. The carrier is not limited to MCC and other naturally-occurring cellulose materials. As is known in the art, materials with a relatively high surface area can be suitable for use as a carrier. The outer water-permeable pouch **20** can be transparent or translucent such that the colored composition is visible to a user of the product **10**. Any combination of alphanumeric characters, designs, patterns, etc. can be dyed on the outer water-permeable pouch and the orientation and type of information illustrated herein are not construed as limiting thereof.

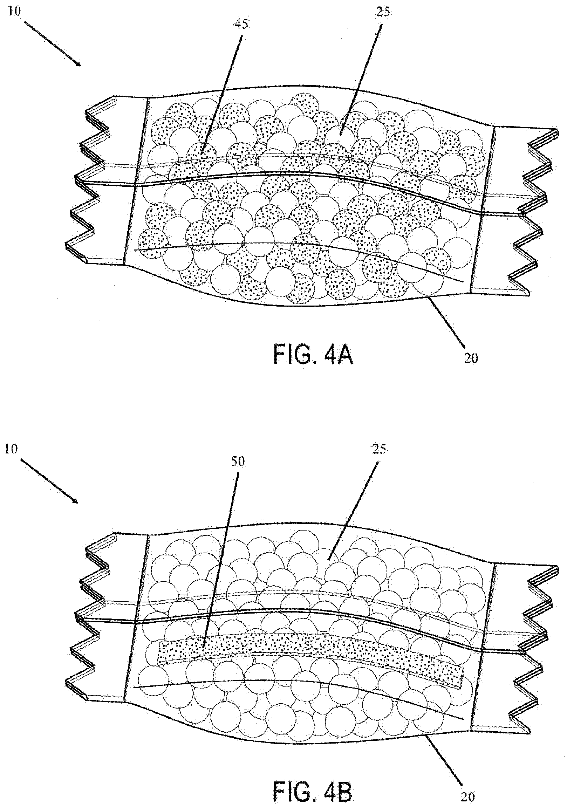

In various illustrative examples, product identifying information can be provided with at least one colored element situated inside the outer water-permeable pouch. The at least one colored element can be any shape and size that fits within the outer water-permeable pouch. The at least one colored element can be formed from an inert ingredient and then blended with the active ingredient and excipients that comprise the nicotine-containing pharmaceutical composition. For example, a cellulose material can be dyed and combined with the nicotine-containing pharmaceutical composition within the pouch. Flavoring agents added to the nicotine-containing pharmaceutical composition can be colored. For example, a menthol or mint flavoring agent can be dyed green and mixed with the nicotine-containing pharmaceutical composition within the pouch. The outer water-permeable pouch can be transparent or translucent such that the at least one colored element is visible to a user of the product. As illustrated in FIG. 4A, for example, various embodiments of the nicotine-containing pharmaceutical product **10** comprise a plurality of colored granules **45** that can be blended with the nicotine-containing pharmaceutical composition **25**. The granules can be any shape and size that fits within the outer water-permeable pouch **20**.

Product identifying information can be provided with at least one product identifying element situated inside the outer water-permeable pouch. As illustrated in FIG. 4B, for example, various embodiments of the nicotine-containing pharmaceutical product **10** comprise at least one product identifying strip **50**. As used herein, strip refers to an identifying element of any width, length and shape that fits into the outer water-permeable pouch **20**. For example, in some embodiments the strip can be rectangular in shape. In various embodiments, the strip can be a spherical rod or a thread-like element. In some embodiments the strip **50** can be inert. In some embodiments, the strip **50** can be flavored. In various embodiments the strip can be dyed or colored, such that the color can indicate product identifying information. In some embodiments, the strip can have product information printed or imprinted on at least one surface of the strip. Any combination of alphanumeric characters, designs, patterns, etc. can be printed or imprinted on a strip and the orientation and type of information disclosed herein are not construed as limiting thereof. The strip can be pre-printed with product indentifying information prior to pouch formation. Alternatively, product identifying information can be printed or imprinted onto a strip during pouch formation. The outer water-permeable pouch **20** can be transparent or translucent such that the at least one product identifying element is visible to a user of the product **10**.

Product identifying information can be provided with at least one product identifying element attached to the outer water-permeable pouch. The product identifying element can be a colored strip for example. As described above, a strip can be any desirable size, color and shape. For example, a thread-like colored strip can provide product identifying information. In some embodiments, the product identifying element can be a strip having product identifying information printed or imprinted on at least one exposed surface of the strip. Any combination of alphanumeric characters, designs, patterns, etc. can be printed or imprinted on a strip and the orientation and type of information disclosed herein are not construed as limiting thereof. Means for attaching the at least one product identifying element to the outer water-permeable pouch can be used such as, but not limited to, adhesive, threading, weaving, heat sealing, etc. In other illustrative examples, at least one product identifying element can be incorporated into the outer water-permeable pouch as the fleece is unwound on a pouching machine. In some embodiments, at least one product identifying element can be incorporated into the nicotine-containing pharmaceutical product after it has been formed and sealed. In some embodiments, at least one product identifying element can be incorporated into the nicotine-containing pharmaceutical product at any point during the product manufacturing process. The at least one product identifying element can be any shape and size that fits onto the outer water-permeable pouch. In some embodiments, the at least one product identifying element can be formed from an inert ingredient. In some embodiments, the at least one product identifying element can be a flavoring agent.

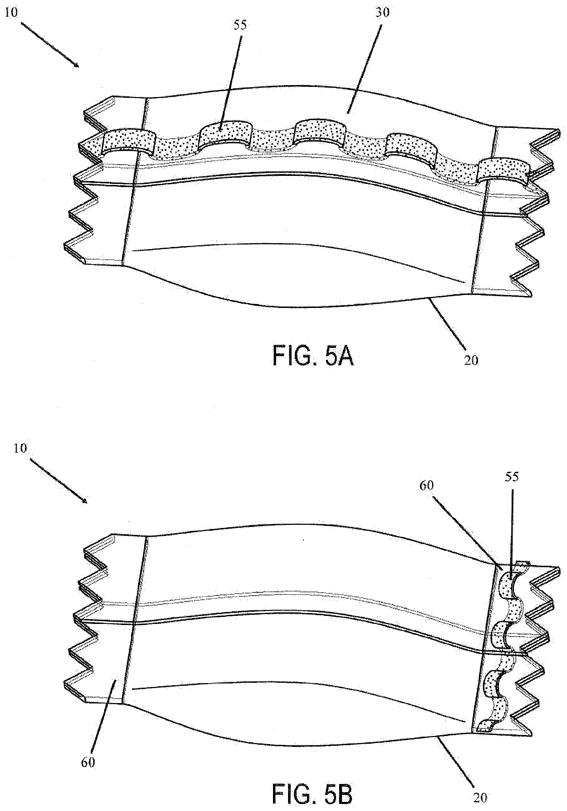

For example, as illustrated in FIGs. 5A and 5B, a colored element that provides product identifying information can be a colored strip or thread of varying widths. For example, as illustrated in FIG. 5A, a colored strip **55** can be woven or threaded through an exposed surface **30** of the outer water-permeable pouch **20**. In some embodiments, the colored strip **55** can be incorporated into the fleece material as the fleece is unwound on a pouching machine. The threaded colored strip **55** can be situated such that the colored strip **55** is visible on an exposed surface **30** of the outer water-permeable pouch **20** once the product **10** has been formed and sealed. In some embodiments, at least one colored strip **55** can be incorporated into the finished product **10**. As illustrated in FIG. 5B, for example, at least one colored strip **55** can be attached to at least one laterally-extending seam **60** of the product **10**. In a preferred embodiment, a colored strip **55** is woven or threaded through the laterally-extending seam **60**. Any number of colored elements can be incorporated into the outer water-permeable pouch and the orientation and type of information illustrated herein are not construed as limiting thereof. Only examples 2A and 2B are according to the invention.

The present invention involves but does not claim, providing a composition that can be used for therapeutic purposes. That is, the composition can be used to treat the cause or symptoms associated with a disease or ailment, or otherwise provide for the well being of the subject to which the composition is administered. The composition can be used as a pharmaceutical composition or as a dietary supplement. The composition incorporates at least nicotine as an active ingredient, and the composition is suitably adapted for oral delivery of that active ingredient. The active ingredient is a compound that can be characterized as a nicotinic compound. Various nicotinic compounds, and methods for their administration, are set forth in US Pat. Pub. No. 2011/0274628 to Borschke. As used herein, "nicotinic compound" or "source of nicotine" often refers to naturally-occurring or synthetic nicotinic compound unbound from a plant material, meaning the compound is at least partially purified and not contained within a plant structure, such as a tobacco leaf. Most preferably, nicotine is naturally-occurring and obtained as an extract from a *Nicotiana* species (e.g., tobacco). The nicotine can have the enantiomeric form S(-)-nicotine, R(+)-nicotine, or a mixture of S(-)-nicotine and R(+)-nicotine. Most preferably, the nicotine is in the form of S(-)-nicotine (e.g., in a form that is virtually all S(-)-nicotine) or a racemic mixture composed primarily or predominantly of S(-)-nicotine (e.g., a mixture composed of about 95 weight parts S(-)-nicotine and about 5 weight parts R(+)-nicotine). Most preferably, the nicotine is employed in virtually pure form or in an essentially pure form. Highly preferred nicotine that is employed has a purity of greater than about 95 percent, more preferably greater than about 98 percent, and most preferably greater than about 99 percent, on a weight basis. Despite the fact that nicotine can be extracted from *Nicotiana* species, it is highly preferred that the nicotine (and the composition and products produced in accordance with the present invention) are virtually or essentially absent of other components obtained from or derived from tobacco.

Nicotinic compounds can include nicotine in free base form, salt form, as a complex, or as a solvate. See, for example, the discussion of nicotine in free base form in US Pat. Pub. No. 2004/0191322 to Hansson. At least a portion of the nicotinic compound can be employed in the form of a resin complex of nicotine, where nicotine is bound in an ion exchange resin, such as nicotine polacrilex. See, for example, US Pat. No. 3,901,248 to Lichtneckert et al. At least a portion of the nicotine can be employed in the form of a salt. Salts of nicotine can be provided using the types of ingredients and techniques set forth in U.S. Pat. Nos. 2,033,909 to Cox et al. and 4,830,028 to Lawson et al., and Perfetti, Beitrage Tabakforschung Int., 12: 43-54 (1983). See, also, US Pub. No. 2011/0268809 to Brinkley et al. Additionally, salts of nicotine have been available from sources such as Pfaltz and Bauer, Inc. and K&K Laboratories, Division of ICN Biochemicals, Inc.

Exemplary pharmaceutically acceptable nicotine salts include nicotine salts of tartrate (e.g., nicotine tartrate and nicotine bitartrate) chloride (e.g., nicotine hydrochloride and nicotine dihydrochloride), sulfate, perchlorate, ascorbate, fumarate, citrate, malate, lactate, aspartate, salicylate, tosylate, succinate, pyruvate, and the like; nicotine salt hydrates (e.g., nicotine zinc chloride monohydrate), and the like. Additional organic acids that can form salts with nicotine include formic, acetic, propionic, isobutyric, butyric, alpha-methylbutyric, isovaleric, beta-methylvaleric, caproic, 2-furoic, phenylacetic, heptanoic, octanoic, nonanoic, oxalic, malonic, and glycolic acid, as well as other fatty acids having carbon chains of up to about 20 carbon atoms.

In many embodiments, the nicotinic compound will be present in multiple forms. For example, the nicotine can be employed within the composition as a mixture of at least two salts (e.g., two different organic acid salts, such as a mixture of nicotine bitartrate and nicotine levulinate), as at least two salts that are segregated within the composition, in a free base form and salt form, in a free base form and a salt form that are segregated within the composition, in a salt form and in a complexed form (e.g., a resin complex such as nicotine polacrilex), in a salt form and in a complexed form that are segregated with in the composition, in a free base form and a complexed form, in a free base form and a complexed form that are segregated within the composition, or the like. As such, each single dosage unit or piece can incorporate at least two forms of nicotine.

A nicotinic compound, in particular as compound such as nicotine, also can be employed in combination with other so-called tobacco alkaloids (i.e., alkaloids that have been identified as naturally occurring in tobacco). For example, nicotine, as employed in accordance with the present invention, can be employed in combination with nornicotine, anatabine, anabasine, and the like, and combinations thereof. See, for example, Jacob et al., Am. J. Pub. Health, 5: 731-736 (1999).

The compositions of the invention possess a form that is pharmaceutically effective and pharmaceutically acceptable. That is, the composition most preferably does not incorporate to any appreciable degree, or does not purposefully incorporate, significant amounts of components of tobacco, other than nicotine. As such, pharmaceutically effective and pharmaceutically acceptable compositions do not include tobacco in parts or pieces, processed tobacco components, or many of the components of tobacco traditionally present within tobacco-containing cigarettes, cigars, pipes, or smokeless forms of tobacco products. Highly preferred compositions that are derived by extracting naturally-occurring nicotine from tobacco include less than 5 weight percent of tobacco components other than nicotine, more often less than about 0.5 weight percent, frequently less than about 0.25 weight percent, and typically are entirely absent or devoid of components of tobacco, processed tobacco components, or components derived from tobacco, other than nicotine, based on the total weight of the composition.

The pharmaceutical compositions of the invention may be conveniently made available in a unit dosage form, whereby formulations may be prepared by any of the methods generally known in the pharmaceutical arts. Such methods of preparation comprise combining (by various methods) an active agent with a suitable carrier or other adjuvant, which may consist of one or more ingredients. The combination of the active ingredient with the one or more adjuvants is then physically treated to provide the formulation in a suitable form for delivery (*e*.*g*., formed into granules for inclusion in a pouch).

The nicotine-containing pharmaceutical compositions of the invention can incorporate various pharmaceutically acceptable excipients. By "pharmaceutically acceptable excipient" is meant an excipient that can be used to facilitate the storage, administration, and/or the healing effect of an active agent (e.g., a nicotinic compound). The excipients are pharmaceutically acceptable in the sense of being compatible with the other ingredients of the formulation and not unduly deleterious to the recipient thereof; and they may also reduce any undesirable side effects of the active agent. See, Wang et al., J. Parent. Drug Assn., 34(6): 452-462 (1980). Exemplary pharmaceutical excipients suitable for use in the compositions according to the invention are listed in Remington: The Science & Practice of Pharmacy, 21st ed., Lippincott Williams & Wilkins (2006); in the Physician's Desk Reference, 64th ed., Thomson PDR (2010); and in Handbook of Pharmaceutical Excipients, 6th ed., Eds. Raymond C. Rowe et al., Pharmaceutical Press (2009).

The various excipients can vary, and the selection and amount of each excipient can depend upon factors such as the ultimate form and function of product that is desired. See, for example, the types of ingredients, relative amounts and combinations of ingredients, nicotine-containing formulations and preparation processes for nicotine-containing products set forth in US Pat. Nos. 5,512,306 to Carlsson et al.; 5,525,351 to Dam; 5,549,906 to Santus; 5,711,961 to Reiner et al.; 5,811,126 to Krishnamurthy; 5,939,100 to Albrechtsen et al.; 6,024,981 to Khankari et al.; 6,083,531 to Humbert-Droz et al.; 6,090,401 to Gowan, Jr. et al.; 6,110,495 to Dam; 6,248,760 to Wilhelmsen; 6,280,761 to Santus; 6,426,090 to Ream et al.; 6,569,463 to Patel et al.; 6,583,160 to Smith et al.; 6,585,997 to Moro et al.; 6,676,959 to Andersson et al.; 6,893,654 to Pinney et al.; 7,025,983 to Leung et al. and 7,163,705 Johnson et al.; US Pat. Pub. Nos. 2003/0176467 to Andersson et al.; 2003/0235617 to Martino et al.; 2004/0096501 to Vaya et al.; 2004/0101543 to Liu et al.; 2004/0191322 to Hansson; 2005/0053665 to Ek et al.; 2005/0123502 to Chan et al.; 2008/0038209 to Andersen et al.; 2008/0286341 to Andersson et al.; 2009/0023819 to Axelsson; 2009/0092573 to Andersen; 2010/0004294 to Axelsson et al.; 2010/0061940 to Axelsson et al.; and 2011/0268809 to Brinkley et al.

Representative types of excipients that are particularly useful for the manufacture of nicotine-containing products include fillers or carriers for active ingredients (e.g., calcium polycarbophil, microcrystalline cellulose, cornstarch, silicon dioxide or calcium carbonate), thickeners, film formers and binders (e.g., hydroxypropyl cellulose, hydroxypropyl methylcellulose, acacia, sodium alginate, xanthan gum and gelatin), buffers and pH control agents (e.g., magnesium oxide, magnesium hydroxide, potassium carbonate, sodium carbonate, potassium bicarbonate, sodium bicarbonate, or mixtures thereof), antiadherents (e.g., talc), glidants (e.g., colloidal silica), natural or artificial sweeteners (e.g., saccharin, acesulfame K, aspartame, sucralose, isomalt, lactose, mannitol, sorbitol, xylitol and sucrose), humectants (e.g., glycerin), preservatives and antioxidants (e.g., sodium benzoate and ascorbyl palmitate), surfactants (e.g., polysorbate 80), natural or artificial flavors (e.g., mint, cinnamon, cherry or other fruit flavors), dyes or pigments (e.g., titanium dioxide or D&C Yellow No. 10), and lubricants or processing aids (e.g., calcium stearate or magnesium stearate). Certain types of nicotine-containing products also can have outer coatings composed of ingredients capable of providing acceptable outer coatings
(e.g., an outer coating can be composed of ingredients such as carnauba wax, and pharmaceutically acceptable forms of shellacs, glazing compositions and surface polish agents).

Representative compositions incorporating nicotine as an active ingredient can have various types of formats and configurations, and as a result, the character, nature, behavior, consistency, shape, form, size and weight of the composition can vary. The general nature of a representative composition can be soft or hard to the touch, or of intermediate softness or hardness; and as such, the composition can be considered to be malleable, flexible, chewy, resilient, brittle, or the like. When administered orally, various components of the product can be considered to be readily dispersible or slow to disperse, or those various components can dissolve at varying rates (e.g., from relatively fast to relatively slow). As a result, for compositions ingested by insertion in the mouth of the human subject, the release rate of active ingredient during use of the product can vary from relatively fast to relatively slow, depending upon factors such as the design of the product and the use of product by the subject using that product. See also, by way of example, the types of products proposed in US Pat. No. 5,147,654 to Place et al.

Formulations of the present invention may include short-term, rapid-onset, rapid-offset, controlled release, sustained release, delayed release, and pulsatile release formulations, providing the formulations achieve administration of active ingredient. See, also, Remington's Pharmaceutical Sciences, 18th ed.; Mack Publishing Company, Eaton, Pennsylvania, (1990).

Solid dosage forms may be formulated so as to provide a delayed release of the active agent (i.e., the nicotinic compound), such as by application of a coating. Delayed release coatings are known in the art, and dosage forms containing such may be prepared by any known suitable method. Such methods generally involve application of a delayed release coating composition after preparation of the solid dosage form (e.g., a tablet or caplet). Application of the coating can be by methods such as airless spraying, fluidized bed coating, use of a coating pan, or the like. Materials for use as a delayed release coating can be polymeric in nature, such as cellulosic material (e.g., cellulose butyrate phthalate, hydroxypropyl methylcellulose phthalate, and carboxymethyl ethylcellulose), and polymers and copolymers of acrylic acid, methacrylic acid, and esters thereof.

Solid dosage forms according to the present invention may also be sustained release (i.e., releasing the active agent over a prolonged period of time), and may or may not also be delayed release. Sustained release formulations are known in the art and are generally prepared by dispersing the active ingredient within a matrix of a gradually degradable or hydrolyzable material, such as an insoluble plastic, a hydrophilic polymer, or a fatty compound. Alternatively, a solid dosage form may be coated with such a material.

The manners and methods used to formulate and manufacture the composition can vary. Typical conditions associated with manufacture of pharmaceutical types of products include control of heat and temperature (i.e., the degree of heat to which the various ingredients are exposed during manufacture and the temperature of the manufacturing environment), moisture content (e.g., the degree of moisture present within individual ingredients and within the final composition), humidity within the manufacturing environment, atmospheric control (e.g., nitrogen atmosphere), airflow experienced by the various ingredients during the manufacturing process, and other similar types of factors. Additionally, various process steps involved in product manufacture can involve selection of certain solvents and processing aids, use of heat and radiation, refrigeration and cryogenic conditions, ingredient mixing rates, and the like. The manufacturing conditions also can be controlled due to selection of the form of various ingredients (e.g., solid, liquid, or gas), particle size or crystalline nature of ingredients of solid form, concentration of ingredients in liquid form, or the like. Ingredients can be processed into the desired composition by techniques such as extrusion, compression, spraying, and the like.

A particularly preferred type of a representative composition incorporating nicotine as an active ingredient, and that provides nicotine in a non-inhalable form, has the form of a pouch or sachet type of product. See, for example, the types of pouch materials and nicotine-containing formulations set forth in US Pat. Pub. No. 2009/0293895 to Axelsson et al. See also, for example, the types of pouch materials and pouch manufacturing techniques (e.g., pouch filling and sealing techniques) set forth in US Pat. Pub. No. 2010/0018539 to Brinkley et al., which is incorporated herein by reference. The amount of composition contained within each pouch can vary. For example, a representative pouch product generally contains at least about 75 mg, often at least about 100 mg, and frequently at least about 150 mg, of composition according to the invention; while the amount of composition contained in a single representative pouch generally does not exceed about 500 mg, often does not exceed about 400 mg, and frequently does not exceed about 300 mg.

The amount of active ingredient within the overall composition can vary. For a composition intended for oral consumption by insertion into the mouth of the subject the amount of nicotine within each dosage piece or unit typically is at least about 0.5 mg, generally is at least 1 mg, often is at least about 1.5 mg, and frequently is at least about 2 mg; while the amount of nicotine within each piece typically does not exceed about 10 mg, generally does not exceed about 8 mg, often does not exceed about 6 mg, and frequently does not exceed about 5 mg, calculated as nicotine base. Exemplary types of such products can incorporate about 2 mg, about 2.5 mg, about 3 mg, about 3.5 mg and about 4 mg of nicotine per piece or unit, calculated as nicotine base.

The dose of active ingredient (i.e., all the various nicotine forms) is that amount effective to treat some symptoms of, or prevent occurrence of the symptoms of, the condition, disease, or disorder from which the subject or patient suffers. By "effective amount", "therapeutic amount" or "effective dose" is meant that amount sufficient to elicit the desired pharmacological or therapeutic effects, thus resulting in effective prevention or treatment of the condition, disease, or disorder. Thus, an effective amount of active ingredient is an amount sufficient to enter relevant regions of the body (e.g., including passing across the blood-brain barrier of the subject), to bind to relevant receptor sites in the CNS and PNS of the subject, and/or to elicit neuropharmacological effects (e.g., elicit neurotransmitter secretion, thus resulting in effective prevention or treatment of the condition, disease, or disorder). Prevention of the disorder is manifested, for example, by delaying the onset of the symptoms of the condition, disease, or disorder. Treatment of the disorder is manifested by, for example, a decrease in the symptoms associated with the condition, disease, or disorder or an amelioration of the reoccurrence of the symptoms thereof.

For compositions of the present invention, the intended daily dose of the active ingredient can vary. The overall dose of active ingredient can depend upon factors such as the weight of the subject ingesting the composition, the type of condition, disease, or disorder being treated, the state or severity of the condition, disease, or disorder being treated, the desired pharmacological effect, or other such factors. Typically, the amount of nicotine active ingredient, calculated as nicotine base, administered to a subject per day is at least about 2 mg, often is at least about 4 mg, and frequently is at least about 10 mg. Typically, the amount of nicotine active ingredient administered to a subject per day does not exceed about 60 mg, often does not exceed about 50 mg, and frequently does not exceed about 40 mg. See also, for example, the types of dosing regimens and administration techniques set forth in US Pat. Nos. 5,593,684 to Baker et al. and 6,660,754 to Kyle et al.; and US Pat. Pub. Nos. 2004/0006113 to Sachs; 2005/0214229 to Pinney et al.; 2008/0124283 to Andersen and 2009/0293895 to Axelsson et al.

The compositions of the present invention can be used for treatment of a wide variety of conditions, diseases, and disorders responsive to stimulation of one or more types of nicotinic acetylcholinergic receptors (nAChRs). The compositions can be used to treat those types of conditions, diseases, and disorders that have been reported to be treatable through the use or administration of nicotine as an agonist of nAChRs, such as neurodegenerative diseases, behavioral disorders, cognitive disorders and cognitive affective disorders. As such, the compositions can be used to treat various CNS conditions, diseases, and disorders, and the compositions also can be used as nicotine-containing products, such as smoking cessation aids (i.e., as components of NRT).

Various manufacturing apparatuses and methods can be used to create a nicotine-containing pharmaceutical product described herein. For example, US Publication No. 2012/0055493 to Novak, III et al., relates to an apparatus and process for providing pouch material formed into a tube for use in the manufacture of smokeless tobacco products, wherein the pouch material tube has one or more objects (e.g., rupturable capsules, pellets, strips, strands, or combinations thereof) disposed along its length such that, when the pouch material tube is subdivided into discrete pouch portions, each pouch portion includes at least one of such objects. Similar apparatuses that incorporate equipment for supplying a continuous supply of a pouch material (e.g., a pouch processing unit adapted to supply a pouch material to a continuous tube forming unit for forming a continuous tubular member from the pouch material) can be used to create a nicotine-containing pharmaceutical product. Representative equipment for forming such a continuous tube of pouch material is disclosed, for example, in U.S. Patent Application Publication No. US 2010/0101588 to Boldrini et al. The apparatus further includes equipment for supplying nicotine-containing pharmaceutical material to the continuous tubular member such that, when the continuous tubular member is subdivided and sealed into discrete pouch portions, each pouch portion includes a nicotine-containing pharmaceutical charge. Representative equipment for supplying the filler material is disclosed, for example, in U.S. Patent Application Publication No. US 2010/0018539 to Brinkley. The apparatus may include, for instance, an object insertion unit for inserting the objects into the continuous tubular member. In some instances, the apparatus may include a subdividing unit for subdividing the continuous tubular member into individual pouch portions and, once subdivided into the individual pouch portions, may also include a sealing unit for sealing at least one of the ends of each pouch portion. In other instances, the continuous tubular member may be sealed into individual pouch portions with a sealing unit and then, once the individual pouch portions are sealed, the continuous tubular member may be subdivided into discrete individual pouch portions by a subdividing unit subdividing the continuous tubular member between the sealed ends of serially-disposed pouch portions. Still in other instances, sealing (closing) of the individual pouch portions of the continuous tubular member may occur substantially concurrently with the subdivision thereof, using a closing and dividing unit.

An exemplary apparatus for manufacturing an oral pouch product incorporating one or more objects is illustrated in FIGS. 1-5 of U.S. Publication No. 2012/0055493 to Novak, III et al.; however, this apparatus is used in a generic and descriptive sense only and not for purposes of limitation. It should also be appreciated that the following manufacturing process and related equipment is not limited to the process order described below. In various embodiments of the present invention, an apparatus similar to that described in U.S. Publication No. 2012/0055493 can be configured to removably receive a first bobbin on an unwind spindle assembly, the first bobbin having a continuous length of a material, such as a pouch material, wound thereon. When the first bobbin is engaged with the apparatus, the pouch material can be routed from the first bobbin to a forming unit configured to form a continuous supply of the pouch material into a continuous tubular member defining a longitudinal axis. Product identifying information can be provided on or incorporated within the continuous length of pouch material before forming the continuous tubular member or even in a secondary offline manufacturing process prior to beginning the pouch formation process. In some embodiments, product identifying information can be provided at any point in the pouch manufacturing process. Product identifying information can be provided, for example, using printing, imprinting, dyeing or weaving equipment known in the art, as taught herein.

As such, as the pouch material is unwound from the first bobbin, the pouch material can be directed around an arrangement of roller members, otherwise referred to herein as a dancer assembly. A forming unit can be configured to cooperate with the first bobbin and the dancer assembly to take up slack in the pouch material and to maintain a certain amount of longitudinal tension on the pouch material as the pouch material is unwound from the first bobbin and fed to the forming unit, for example, by a drive system. One of ordinary skill in the art will appreciate that, between the first bobbin and the forming unit, the pouch material can be supported, routed, and/or guided by a suitably aligned series of any number of, for example, idler rollers, guideposts, air bars, turning bars, guides, tracks, tunnels, or the like, for directing the pouch material along the desired path. Typical bobbins used by conventional automated pouch making apparatuses often contain a continuous strip of pouch material of which the length may vary. As such, the apparatus described herein can be configured so as to handle bobbins of that type and size.

The forming unit can include one or more roller members configured to direct the pouch material about a hollow shaft such that the continuous supply of the pouch material can be formed into a continuous tubular member. The forming unit can include a sealing device configured to seal, fix, or otherwise engage lateral edges of the pouch material to form a longitudinally-extending seam, thereby forming a longitudinally-extending continuous tubular member. An imprinting unit is configured to imprint product identifying information onto the longitudinally-extending seam as the lateral edges of the pouch material are sealed. Thereby, the imprinting unit provides product identifying information, as well as contribute to the sealing of the pouch.

Product identifying information can be printed, imprinted, dyed or attached to the longitudinally-extending continuous tubular member. Equipment known in the art can be configured to provide product identifying information corresponding to the nicotine-containing pharmaceutical composition inserted into the tubular member in subsequent steps of a method of forming an embodiment of the pouch product disclosed herein.

In various embodiments, a nicotine-containing pharmaceutical insertion unit can be configured to introduce nicotine containing pharmaceutical material into the continuous tubular member through the hollow shaft. In some embodiments a product identifying object insertion unit can be configured to introduce objects into the continuous tubular member, also through the hollow shaft. The nicotine containing pharmaceutical insertion unit may be directly or indirectly engaged with the hollow shaft. Further, the product identifying object insertion unit may be directly or indirectly engaged with the hollow shaft.

A leading edge or end (also referred to as a laterally-extending seam) of the continuous tubular member can be closed/sealed such that a charge of nicotine containing pharmaceutical material and any objects introduced by the nicotine containing pharmaceutical insertion unit and optional object insertion unit, respectively, are contained within the continuous tubular member proximate to the leading end. The leading end can be closed/sealed via a closing and dividing unit configured to close/seal a first portion of the continuous tubular member to form the closed leading end of a pouch member portion. In various embodiments, an imprinting unit can be configured to imprint product identifying information onto the leading edge or end (also referred to as a laterally-extending seam) as the pouch member portion is sealed. Thereby, the imprinting unit can provide product identifying information, as well as contribute to the sealing of the pouch. The closing and dividing unit can also be configured to form a closed trailing edge or end of a previous pouch member portion. In this regard, the closing and dividing unit can also be configured to close a second portion of the continuous tubular member to form the closed trailing end of the pouch member portion. The closing and dividing unit can also be configured to form a closed leading edge of a subsequent pouch member portion. That is, the closing and dividing unit can be configured to close the trailing end of one pouch member portion while simultaneously closing the leading end of a subsequent pouch member portion formed from the continuous tubular member. In this regard, the closing and dividing unit can close the ends, by heat-sealing, a suitable adhesive, or other suitable sealing mechanism.

Furthermore, as illustrated in FIGS. 20-22 of U.S. Publication No. 2012/0055493, the closing and dividing unit can be configured to divide the continuous tubular member, between the closed trailing end and the closed leading end of serially-disposed pouch member portions, along the longitudinal axis of the continuous tubular member, and into a plurality of discrete pouch member portions such that each discrete pouch member portion includes a portion of the nicotine-containing pharmaceutical material from the nicotine-containing pharmaceutical insertion unit and at least one form of product identifying information is provided on and/or within each portion. In this regard, the closing and dividing unit can include a blade, heated wire, or other cutting arrangement for severing the continuous tubular member into discrete pouch member portions. For example, the closing and dividing unit can include first and second arm members configured to interact to close and divide the continuous tubular member.

Machines can be situated and or calibrated such that product identifying information can be printed, imprinted, dyed or attached at any location on the nicotine-containing pharmaceutical product, or inserted within each nicotine-containing pharmaceutical product. The product identifying information can be provided at any step during the product manufacturing process. In general, product identifying information can be provided on or attached to each discrete pouch member portion after the continuous tubular member has been sealed and divided.

In operation, a charge of nicotine containing pharmaceutical material (i.e., an amount suitable for an individual pouch member portion) can supplied to the pouch member portion by a nicotine containing pharmaceutical insertion unit after a leading end has been closed, but prior to the closing of a trailing end. Similarly, one or more product identifying objects can be supplied to the pouch member portion by a product identifying object insertion unit after the leading end has been closed, but prior to the closing of the trailing end. After receiving the charge of nicotine-containing pharmaceutical material and the optional one or more product identifying objects, the discrete individual pouch member portion can be formed by closing the trailing end and severing the closed pouch member portion from the continuous tubular member such that an individual smokeless nicotine-containing pharmaceutical product is formed.

In some instances, the apparatus can be configured to produce approximately 300 pouch member portions per minute. A conveyor assembly can be provided proximate to the closing and dividing unit such that, after being severed from the continuous tubular member, each individual pouch member portion is received by the conveyor assembly and transported away from the apparatus to, for example, a storage bin or container. In some instances, each individual pouch member portion can be transported to a counting device capable of counting and depositing a predetermined quantity of individual pouch member portions into, for example, a packaging container. A computer device can provide a signal to the nicotine containing pharmaceutical insertion unit, and/or the object insertion unit to indicate when the charge of nicotine containing pharmaceutical material and/or the object(s) should be directed into the continuous tubular member. That is, the computer device can be used to control the timing of the insertion of the nicotine-containing pharmaceutical material and product identifying objects.

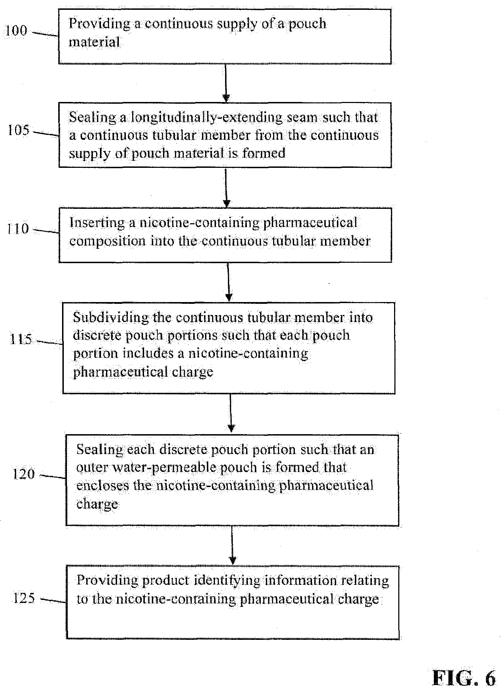

As illustrated in FIG. 6, for example, a method of manufacturing a nicotine-containing pharmaceutical pouch product can comprise a number of general, non-limiting operations that can be performed in any desirable order. At operation **101**, a continuous supply of a pouch material can be provided. At operation **105**, the pouch material is formed into a continuous tubular member by sealing the lateral edges of the pouch material such that a longitudinally-extending seam is formed. At operation **110**, a nicotine-containing pharmaceutical material can be inserted into the continuous tubular member. At operation **115**, the continuous tubular member can be subdivided at predetermined intervals so as to form a plurality of pouch member portions, wherein each pouch member portion includes nicotine-containing pharmaceutical composition. At operation **120**, each discrete pouch portion can be entirely sealed such that an outer water-permeable pouch is formed that encloses the nicotine-containing pharmaceutical composition. At operation **125**, product identifying information relating to the nicotine-containing pharmaceutical charge within each discrete pouch portion can be provided to each pouch portion. Product identifying information can be provided by printing, imprinting, dying or otherwise marking the pouch material and/or the nicotine-containing pharmaceutical composition, for example. In some embodiments, each pouch member further comprises at least one product identifying object therein. In some embodiments, each pouch member further comprises at least one product identifying object on an outer surface of the pouch member. Accordingly, aspects of the present disclosure are particularly configured to provide discrete nicotine-containing pharmaceutical pouch products comprising product identifying information and nicotine-containing pharmaceutical composition. The operations described and the order of the method steps illustrated herein are not construed as limiting thereof.

Although the invention is directed to nicotine-containing pharmaceutical compositions, product identifying information can be provided in the same manner described herein for more traditional tobacco products, including smokeless tobacco products provided in pouches, such as snus type products.

Many modifications and other embodiments of the invention will come to mind to one skilled in the art to which this invention pertains having the benefit of the teachings presented in the foregoing description. Therefore, it is to be understood that the invention is not to be limited to the specific embodiments disclosed and that modifications and other embodiments are intended to be included within the scope of the appended claims. Although specific terms are employed herein, they are used in a generic and descriptive sense only and not for purposes of limitation.

## Claims

1. A nicotine-containing pharmaceutical product configured for insertion into the mouth of a user of that product, comprising:
an outer water-permeable pouch containing a nicotine-containing pharmaceutical composition; and
product identifying information relating to the nicotine-containing pharmaceutical composition presented such that said product identifying information is discernible to a user of the product upon visual inspection of the product and which enables the user to differentiate multiple nicotine-containing pharmaceutical products, the product identifying information comprising one or more of alphanumeric characters and designs associated with the nicotine-containing pharmaceutical product, wherein the product identifying information is imprinted on at least one of a longitudinally-extending seam and a laterally-extending seam of the outer water-permeable pouch, and
wherein patterned roller bars are used to press at least one of the longitudinally-extending seam and the laterally-extending seam of the outer water-permeable pouch during sealing to assist in marking the outer water-permeable pouch as a seam is pressed, such that the imprinted product identifying information contributes to the sealing of at least one of the longitudinally-extending seam and the laterally-extending seam of the outer water-permeable pouch.

2. The nicotine-containing pharmaceutical product of claim 1, wherein the product identifying information is selected from the group consisting of product brand, company name, corporate logo, corporate brand, a marketing message, product strength, active ingredient, product manufacture date, product expiration date, product flavor, product pharmaceutical release profile, weight, product code, other product differentiating markings, and combinations thereof.

3. The nicotine-containing pharmaceutical product of claim 1 or 2, wherein the imprinted product identifying information identifies the strength of the nicotine-containing pharmaceutical composition.

4. A method for manufacturing a nicotine-containing pharmaceutical product configured for insertion into the mouth of a user of that product, the method comprising:
providing a continuous supply of a pouch material;
engaging lateral edges of the pouch material such that a longitudinally-extending seam is formed;
sealing the longitudinally-extending seam such that a continuous tubular member is formed from the continuous supply of pouch material;
inserting a nicotine-containing pharmaceutical composition into the continuous tubular member;
subdividing the continuous tubular member into discrete pouch portions such that each pouch portion includes a nicotine-containing pharmaceutical charge;
sealing a leading and an end edge of each discrete pouch portion such that an outer water-permeable pouch is formed that encloses the nicotine-containing pharmaceutical charge;
providing product identifying information relating to the nicotine-containing pharmaceutical charge such that said product identifying information is discernible to a user of the product upon visual inspection of the product and which enables the user to differentiate multiple nicotine-containing pharmaceutical products, the product identifying information comprising one or more of alphanumeric characters and designs associated with the nicotine-containing pharmaceutical product, wherein the product identifying information is imprinted on at least one of the longitudinally-extending seam and the laterally-extending seam of the outer water-permeable pouch, and
wherein patterned roller bars are used to press at least one of the longitudinally-extending seam and the laterally-extending seam of the outer water-permeable pouch during sealing of each discrete pouch portion to assist in marking the outer water-permeable pouch as a seam is pressed, such that the imprinted product identifying information contributes to the sealing of at least one of the longitudinally-extending seam and the laterally-extending seam of the outer water-permeable pouch.

5. The method of claim 4, wherein the product identifying information is selected from the group consisting of product brand, company name, corporate logo, corporate brand, a marketing message, product strength, active ingredient, product manufacture date, product expiration date, product flavor, product pharmaceutical release profile, weight, product code, other product differentiating markings, and combinations thereof.

6. The method of claim 4 or 5, wherein the imprinted product identifying information identifies the strength of the nicotine-containing pharmaceutical composition.

## Patentansprüche

1. Nikotin enthaltendes pharmazeutisches Produkt, das zum Einführen in den Mund eines Benutzers dieses Produktes ausgelegt ist, umfassend:
einen äußeren wasserdurchlässigen Beutel, enthaltend eine Nikotin enthaltende pharmazeutische Zusammensetzung; und
Informationen zur Produktidentifikation in Bezug auf die Nikotin enthaltende pharmazeutische Zusammensetzung, die so präsentiert sind, dass die Informationen zur Produktidentifikation für einen Benutzer des Produktes bei Sichtprüfung des Produktes wahrnehmbar sind, und die den Benutzer befähigen, mehrere Nikotin enthaltende pharmazeutische Produkte zu unterscheiden, wobei die Informationen zur Produktidentifikation ein oder mehrere alphanumerische Zeichen und Muster umfassen, die mit dem Nikotin enthaltenden pharmazeutischen Produkt assoziiert sind, wobei die Informationen zur Produktidentifikation auf einem sich längs erstreckenden Rand und/oder auf einem sich seitlich erstreckenden Rand des äußeren wasserdurchlässigen Beutels eingeprägt sind, und
wobei gemusterte Rollen verwendet werden, um den sich längs erstreckenden Rand und/oder den sich seitlich erstreckenden Rand des äußeren wasserdurchlässigen Beutels während des Versiegelns zu pressen, um zur Markierung des äußeren wasserdurchlässigen Beutels beizutragen während ein Rand gepresst wird, sodass die eingeprägten Informationen zur Produktidentifikation zur Versiegelung des sich längs erstreckenden Randes und/oder des sich seitlich erstreckenden Randes des äußeren wasserdurchlässigen Beutels beitragen.

2. Nikotin enthaltendes pharmazeutisches Produkt nach Anspruch 1, wobei die Informationen zur Produktidentifikation ausgewählt sind aus der Gruppe bestehend aus Produktmarke, Unternehmensname, Firmenlogo, Firmenmarke, einer Marketingbotschaft, Produktstärke, aktivem Inhaltsstoff, Produktherstellungsdatum, Produktverfallsdatum, Produktaroma, pharmazeutischem Freisetzungsprofil des Produkts, Gewicht, Produktcode oder anderen das Produkt unterscheidenden Markierungen, und Kombinationen hiervon.

3. Nikotin enthaltendes pharmazeutisches Produkt nach Anspruch 1 oder 2, wobei die eingeprägten Informationen zur Produktidentifikation die Stärke der Nikotin enthaltenden pharmazeutischen Zusammensetzung identifizieren.

4. Verfahren zur Herstellung eines Nikotin enthaltenden pharmazeutischen Produktes, das zum Einführen in den Mund eines Benutzers dieses Produktes ausgelegt ist, wobei das Verfahren umfasst:
Bereitstellen einer kontinuierlichen Zuführung eines Beutelmaterials;
Inkontaktbringen von seitlichen Rändern des Beutelmaterials, sodass ein sich längs erstreckender Rand gebildet wird;
Versiegeln des sich längs erstreckenden Randes, sodass aus der kontinuierlichen Zuführung von Beutelmaterial ein kontinuierliches schlauchförmiges Element gebildet wird;
Einführen einer Nikotin enthaltenden pharmazeutischen Zusammensetzung in das kontinuierliche schlauchförmige Element;
Unterteilen des kontinuierlichen schlauchförmigen Elements in separate Beutelabschnitte, sodass jeder Beutelabschnitt eine Nikotin enthaltende pharmazeutische Füllung umfasst;
Versiegeln eines vorderen Randes und eines hinteren Randes von jedem einzelnen Beutelabschnitt, sodass ein äußerer wasserdurchlässiger Beutel gebildet wird, der die Nikotin enthaltende pharmazeutische Füllung einschließt;
Bereitstellen von Informationen zur Produktidentifikation in Bezug auf die Nikotin enthaltende pharmazeutische Füllung, sodass die Informationen zur Produktidentifikation für einen Benutzer des Produktes bei Sichtprüfung des Produktes wahrnehmbar sind, und die den Benutzer befähigen, mehrere Nikotin enthaltende pharmazeutische Produkte zu unterscheiden, wobei die Informationen zur Produktidentifikation ein oder mehrere alphanumerische Zeichen und Muster umfassen, die mit dem Nikotin enthaltenden pharmazeutischen Produkt assoziiert sind, wobei die Informationen zur Produktidentifikation auf einem sich längs erstreckenden Rand und/oder auf einem sich seitlich erstreckenden Rand des äußeren wasserdurchlässigen Beutels eingeprägt sind, und
wobei gemusterte Rollen verwendet werden, um den sich längs erstreckenden Rand und/oder den sich seitlich erstreckenden Rand des äußeren wasserdurchlässigen Beutels während des Versiegelns von jedem separatem Beutelabschnitt zu pressen, um zur Markierung des äußeren wasserdurchlässigen Beutels beizutragen während ein Rand gepresst wird, sodass die eingeprägten Informationen zur Produktidentifikation zur Versiegelung des sich längs erstreckenden Randes und/oder des sich seitlich erstreckenden Randes des äußeren wasserdurchlässigen Beutels beitragen.

5. Verfahren nach Anspruch 4, wobei die Informationen zur Produktidentifikation ausgewählt sind aus der Gruppe bestehend aus Produktmarke, Unternehmensname, Firmenlogo, Firmenmarke, einer Marketingbotschaft, Produktstärke, aktivem Inhaltsstoff, Produktherstellungsdatum, Produktverfallsdatum, Produktaroma, pharmazeutischem Freisetzungsprofil des Produkts, Gewicht, Produktcode oder anderen das Produkt unterscheidenden Markierungen, und Kombinationen hiervon.

6. Verfahren nach Anspruch 4 oder 5, wobei die eingeprägten Informationen zur Produktidentifikation die Stärke der Nikotin enthaltenden pharmazeutischen Zusammensetzung identifizieren.

## Revendications

1. Produit pharmaceutique contenant de la nicotine configurée pour être inséré dans la bouche d'un utilisateur de ce produit, comprenant :
un sachet extérieur perméable à l'eau renfermant une composition pharmaceutique contenant de la nicotine ; et
des informations d'identification du produit concernant la composition pharmaceutique contenant de la nicotine présentées de manière à ce que lesdites informations d'identification du produit soient reconnaissables par un utilisateur du produit lors de l'inspection visuelle du produit, et qui permettent à l'utilisateur de différencier plusieurs produits pharmaceutiques contenant de la nicotine, les informations d'identification du produit comprenant un ou plusieurs caractères alphanumériques et motifs associés au produit pharmaceutique contenant de la nicotine, les informations d'identification du produit étant imprimées sur au moins une soudure parmi une soudure s'étendant longitudinalement et une soudure s'étendant latéralement du sachet extérieur perméable à l'eau, et
dans lequel des barres à galets à motifs sont utilisées pour presser au moins une soudure parmi la soudure s'étendant longitudinalement et la soudure s'étendant latéralement du sachet extérieur perméable à l'eau au cours du scellement pour faciliter le marquage du sachet extérieur perméable à l'eau lorsque la soudure est pressée, de telle sorte que les informations d'identification du produit imprimées contribuent au scellement d'au moins une soudure parmi la soudure s'étendant longitudinalement et la soudure s'étendant latéralement du sachet extérieur perméable à l'eau.

2. Produit pharmaceutique contenant de la nicotine selon la revendication 1, dans lequel les informations d'identification du produit sont choisies dans le groupe constitué de la marque du produit, du nom de la société, du logo d'entreprise, de la marque d'entreprise, d'un message marketing, de la concentration du produit, du principe actif, de la date de fabrication du produit, de la date d'expiration du produit, de l'arôme du produit, du profil de libération pharmaceutique du produit, du poids, du code produit, de caractéristiques différenciatrices d'autres produits et des combinaisons de ceux-ci.

3. Produit pharmaceutique contenant de la nicotine selon la revendication 1 ou 2, dans lequel les informations d'identification du produit imprimées identifient la concentration de la composition pharmaceutique contenant de la nicotine.

4. Procédé pour fabriquer un produit pharmaceutique contenant de la nicotine configuré pour être inséré dans la bouche d'un utilisateur de ce produit, le procédé comprenant :
un approvisionnement continu en matériau pour sachets ;
la mise en prise des bords latéraux du matériau pour sachets de manière à former une soudure s'étendant longitudinalement ;
le scellement de la soudure s'étendant longitudinalement de manière à former un élément tubulaire continu à partir de l'approvisionnement continu en matériau pour sachets ;
l'insertion d'une composition pharmaceutique contenant de la nicotine dans l'élément tubulaire continu ;
la subdivision de l'élément tubulaire continu en parties de sachet discrètes de manière à ce que chaque partie de sachet comprenne une charge pharmaceutique contenant de la nicotine ;
le scellement d'un bord d'attaque et d'un bord arrière de chaque partie de sachet discrète de manière à former un sachet extérieur perméable à l'eau qui renferme la charge pharmaceutique contenant de la nicotine ;
la fourniture d'informations d'identification du produit concernant la charge pharmaceutique contenant de la nicotine de manière à ce que lesdites informations d'identification du produit soient reconnaissables par un utilisateur du produit lors de l'inspection visuelle du produit, et qui permettent à l'utilisateur de différencier plusieurs produits pharmaceutiques contenant de la nicotine, les informations d'identification du produit comprenant un ou plusieurs caractères alphanumériques et motifs associés au produit pharmaceutique contenant de la nicotine, les informations d'identification du produit étant imprimées sur au moins une soudure parmi la soudure s'étendant longitudinalement et la soudure s'étendant latéralement du sachet extérieur perméable à l'eau, et
dans lequel des barres à galets à motifs sont utilisées pour presser au moins une soudure parmi la soudure s'étendant longitudinalement et la soudure s'étendant latéralement du sachet extérieur perméable à l'eau au cours du scellement de chaque partie discrète de sachet pour faciliter le marquage du sachet extérieur perméable à l'eau lorsqu'une soudure est pressée, de telle sorte que les informations d'identification du produit imprimées contribuent au scellement d'au moins une soudure parmi la soudure s'étendant longitudinalement et la soudure s'étendant latéralement du sachet extérieur perméable à l'eau.

5. Procédé selon la revendication 4, dans lequel les informations d'identification du produit sont choisies dans le groupe constitué de la marque du produit, du nom de la société, du logo d'entreprise, de la marque d'entreprise, d'un message marketing, de la concentration du produit, du principe actif, de la date de fabrication du produit, de la date d'expiration du produit, de l'arôme du produit, du profil de libération pharmaceutique du produit, du poids, du code produit, de caractéristiques différenciatrices d'autres produits et des combinaisons de ceux-ci.

6. Procédé selon la revendication 4 ou 5, dans lequel les informations d'identification du produit imprimées identifient la concentration de la composition pharmaceutique contenant de la nicotine.
